# EUROPEAN PATENT APPLICATION

(11) **EP 1 471 351 A1**
(43) Date of publication of application: **27.10.2004**
(21) Application number: 04007073.2
(22) Date of filing: 24.03.2004
(51) Int. Cl.: G01N 30/86, G01N 33/28, G01N 30/02

(54) **Analytical method for the detection and quantitation of fuel additives**

(30) Priority: 25.04.2003 US 424493
(71) Applicant: ETHYL CORPORATION, Richmond, Virginia 23219-4304 (US)
(72) Inventor: Fedorova, Galina, V., Ricmond, VA 23233 (US)
(74) Representative: Schüssler, Andrea, Dr.

(57) **Abstract**

The identity and concentration of an additive package in a liquid hydrocarbon matrix may be measured by an analytical liquid chromatography analysis. A sample is prepared and passed through a separation means and at least one detector means, and signals are generated that permit identification and quantitative measurement of additives in a liquid hydrocarbon matrix.

## Description

### TECHNICAL FIELD

The present invention relates to methods for determining the presence and concentration of additive packages, components, and concentrates in liquid hydrocarbons using chromatographic analysis.

### BACKGROUND OF THE INVENTION

Prior to the present invention, determining the type or amount of an additive package in a liquid hydrocarbon matrix, such as fuel, was achieved through the addition of markers. Markers that are conventionally incorporated into fuel products include colors or dyes, chemical labels, and radioactive tags. The latter technique is costly, and all of these methods provide only an indirect assessment of the identity and concentration of an additive package, rather than a direct measurement of the additives in the matrix.

For example, U.S. Patent Number 6,312,958 provides a method for marking liquids with fluorescent or radioactive tags, U.S. Patent Number 6,214,624 provides a method for marking additive packages with perfluorocarbons, and U.S. Patent Number 6,482,651 utilizes aromatic esters for marking or tagging additives in petroleum products. These methods are often used to non-visibly characterize fuels containing additives, to prevent fuel distributors from diluting commercially expensive additized fuels with less costly fuels having different additive constituents, a process commonly known as "cross-hauling."

Another approach for detecting cross-hauling is the addition of colored dyes as markers in fuels, as exemplified in U.S. Patent Number 6,007,744. A somewhat quantitative approach is demonstrated in U.S. Patent Number 5,244,809, in which a rough estimate of the concentration of additives is achieved using a light-scattering detector. However, this method could yield inaccurate estimations in at least two ways: first, the result includes all unevaporated material, such as particulate matter and contaminants; second, it does not identify different fuel additive packages and/or mixtures, which prevents proper calibration for quantitative analysis and is therefore susceptible to error. The present invention overcomes previous uncertainties in determining the concentration of fuel additives without reference to a marker's presence, or by any other rough estimation.

In general, a preferred method for quantitating high molecular weight polar components in solution is by high-performance liquid chromatography (HPLC) or gel permeation chromatography (GPC). One advantage offered by these analytical methods is that the process of separation and peak correlation provides reliable determinations of both identity and concentration. However, it is very difficult to use these methods to analyze samples that will not properly elute, or be removed from an analytical separating column due to their chemical nature. The complex nature of fuels containing additive packages had previously prohibited quantitative, chromatographic analytical analysis of multiple samples of fuels. Thus, a need exists for an analytical technique able to identify fuel additive packages and determine their presence quantitatively.

The present invention overcomes this problem and provides an accurate and precise method for identifying and quantitating additive packages in hydrocarbon matrices, such as fuels.

### DETAILED DESCRIPTION OF THE INVENTION

The present invention is directed to a novel method for determining the identity and concentration of additive packages, components, concentrates, or mixtures thereof in liquid hydrocarbon matrices. The present invention overcomes previous difficulties using analytical equipment to perform a quantitative analysis. In an embodiment, the method of the present invention determines the identity and concentration of an additive package in liquid hydrocarbon matrix by:
(1) delivering a sample to an analytical system comprising at least one separation means and at least one detector means, whereby at least one signal is generated by the analytical system; and
(2) comparing at least one signal from a standard of an additive package in a hydrocarbon matrix to the at least one signal from the sample, to thereby determine the identity and/or concentration of an additive package in the sample.

By liquid hydrocarbon matrix herein is meant any fuel, light naphtha, gasoline, kerosene, diesel fuel, jet fuel, turbine combustion fuel oils, gas oils, lubricants, transmission fluids, hydraulic fluids, and the like. Fuels suitable for use in the present invention include hydrocarbonaceous fuels derived from any solid, liquid, or gaseous hydrocarbon material, including but not limited to, crude oil or chemically synthesized hydrocarbons such as diesel fuel, jet fuel, kerosene, low sulfur fuels, synthetic fuels including Fischer-Tropsch fuels, liquid petroleum gas, fuels derived from coal, genetically engineered biofuels and crops and extracts therefrom, unleaded motor and aviation gasolines, and so-called reformulated gasolines which typically contain both hydrocarbons of the gasoline boiling range and fuel-soluble oxygenated blending agents, such as alcohols, ethers and other suitable oxygen-containing organic compounds.

By "diesel fuel" herein is meant one or more fuels selected from the group consisting of diesel fuel, biodiesel, biodiesel-derived fuel, synthetic diesel and mixtures thereof. By "oils" herein is meant a base oil which can be selected from the group consisting of paraffinic, naphthenic, aromatic, poly-alpha-olefins, synthetic esters, and polyol esters, and mixtures thereof.

By "additive package" herein is meant an element or mixture of components added to a liquid hydrocarbon matrix which may include, but are not limited to, dispersants or detergents derived from Mannich condensation products, amines, aliphatic hydrocarbon N-substituted amines, alkylene polyamines, polyetheramines, succinimides, succinamides, polybutene amines, alkanolamines, and hydroxyalkyl-substituted polyamines. The liquid solvents or carrier fluids included in an additive packages may be, but are not limited to, polyether monools, polyols, poly alpha-olefins, mineral oils, polyolefins, aromatic carboxylic acids, and the like. Other additive package components also include rust or corrosion inhibitors, demulsifying agents, metal deactivators, combustion modifiers, octane improvers, emission reducers, friction modifiers, lubricity additives, antioxidants, pour point depressants, multifunctional additives, and the like.

By "separation means" herein is meant columns, beads, filters, frits, plates, and the like for chromatography, normal and reverse phase high-performance liquid chromatography (HPLC), gel permeation chromatography (GPC), capillary chromatography, ion chromatography, thin layer and high pressure thin layer chromatography, among others known now or developed in the future.

By "detector means" herein is meant analytical detectors including but not limited to the use of one or more detectors that may include an ultraviolet (UV) detector, a refractive index detector (RID), light-scattering and evaporative light-scattering detectors, a fluorescence detector, a mass spectrophotometric detector, and the like. By a "signal" herein is meant a detectable, reproducible, or measurable response in conjunction with the use of an analytical detector.

It is known to those skilled in the art of analytical chemistry to employ various chromatographic techniques and equipment to determine the concentration of a particular constituent in a liquid sample. In an embodiment, the sample containing an additive package would be applied to, or forced through, a separation means to separate species dissolved in the solvent. If the sample solution contacts a second solid or liquid phase, the different constituents interact with the other phase to differing degrees due to differences in adsorption, ion-exchange, partitioning, or size. These differences allow the mixture of components to be separated from each other by measuring the different transit or retention times of the solutes through the separation medium.

Liquid chromatography most often separates solutes through an analytical column as a separating means, injecting a solution into a pump that pushes the solution through tubing, column(s), and detector(s). The solution flowing through the analytical system is considered a mobile phase, and the separating means is considered a stationary phase. A signal in the form of a chromatogram may then be detected and used to evaluate unknown samples against those generated by a standard.

In a preferred embodiment, the additive package and the liquid hydrocarbon matrix could be free of conventional markers, chemical labels, radioactive tags, dyes, etc.

In an embodiment, a syringe is used to draw up an aliquot (2 ml) of the liquid hydrocarbon sample containing the additive package. A pre-washed filter is then placed on the end of the syringe and the sample is ejected from the syringe into an HPLC sample vial. By way of example and without limitation, the filter is preferably a 0.01-0.5 micron filter coated with nonreactive materials including, but not limited to fluoropolymer resins, such as polytetrafluoroethylene (PTFE), and the like. The vial is then closed immediately to prevent sample evaporation.

The HPLC equipment is usually equilibrated for at least one hour with a mobile phase solvent, which is preferably tetrahydrofuran (THF), or any solvent of similar or higher polarity that is compatible with the chromatography column material. Representative solvents include, but are not limited to, toluene, ethyl acetate, tetrahydrofuran (THF), chloroform, methyl ethyl ketone (MEK), dichloromethane, dichloroethane, acetone, o-dichlorobenzene (o-DCB), trichlorobenzene (TCB), m-cresol, and o-chlorophenol (o-CP). Depending on the nature of the additives in the package, an aliquot of a dialkyl acetamide solvent may be injected through the analytical system after each sample in order to reduce undesirable sample-column interactions to maintain the optimal condition of the column. The dialkyl acetamide solvent is preferably dimethylacetamide (DMA) or a solvent of similar or higher polarity that is compatible with the separation materials, including, but not limited to, pyridine, n-methyl pyrolidone (NMP), dimethyl sulphoxide (DMSO), and dimethyl formamide (DMF).

In an embodiment, the column or columns used for analysis by HPLC may be, but are not limited to, a set of at least two or three gel permeation chromatography/size exclusion chromatography (GPC/SEC) columns. These columns are preferably 100Å, 3 micron, 300 x 7.5 mm columns connected using, for example, 0.17 mm i.d. stainless steel tubing, preferably cut at minimum lengths. Various types of stationary phase columns are available for use in liquid chromatography and, potentially, could be used for detection of additive packages.

The analytical process and system includes at least one detector, but preferably at least three detectors connected in series, for determining the identity and concentration of an additive package after the sample has passed through or over a separation means. Generally, detectors are used to measure the solute's relative changes in refractive index, ultraviolet/visible (UV/VIS) light, fluorescence, conductivity, mass-spectrometry, and evaporative light scattering, among other comparative techniques.
In an embodiment, the series of detectors of the present invention preferably evaluate the sample or standard in the following sequence: a UV/VIS detector, followed by a fluorescence detector, and optionally leading to a refractive index detector (RID) or an evaporative light scattering detector (ELSD). At the end of the series may also be placed a mass spectrophotometer detector (MSD). When both an ELSD and MSD are used, the flow from the UV/VIS and fluorescence detectors would be split between ELSD and MSD.

In an embodiment, after each sample passes through the system of at least one detector, at least one signal in the form of a chromatogram may be generated by at least one of the detectors. Upon completion of analysis of known standards, a "fingerprint" or representative array of chromatograms may be generated for each additive package representing its interaction with each detector in the series. The array of cumulative "fingerprint" information allowing identification of an additive package may be used to determine whether a particular sample has been contaminated, as this would elicit a different pattern of response than an unadulterated standard. A quantitative determination may also then be made extrapolating against the "fingerprint" generated by the standard, as the ratios of responses would be integrated and calculated by analytical software to yield the concentration of a sample. This determination would be more accurate than estimations that did not rely on separation means, as it correlates the signals from matched standards and samples without interference from contaminants.

In a preferred embodiment, the GPA contains a dispersant, such as but not limited to a polyalkylene-substituted succinimide dispersant, whereby the analytical method can identify the amount of the dispersant(s) present, and by comparison to a database of known dispersants, allow the identification of the source of the dispersant(s).

### EXAMPLE 1

Fifteen hundred gasoline fuel samples were analyzed using the novel methods described herein to determine the presence and concentration of the gasoline package additives (GPAs) known as Ethyl HiTEC® 6476, HiTEC®6421, and other GPAs. A 2 ml aliquot of each sample of fuel was drawn into a syringe, and a 0.2 micron PTFE filter was placed on the end of the syringe. The fuel sample was then ejected through the filter into an HPLC vial. A crimp-top lid with a Teflon®-rubber septum was immediately placed on the vial to seal it and prevent sample evaporation.

The HPLC equipment was equilibrated by priming it with a mobile phase equilibrating solvent for approximately 1 hour prior to sample analysis, at a flow rate of 0.05-2.5 ml/min, preferably 0.3 ml/min when using two separation columns, and preferably 0.6 ml/min when using three separation columns. The mobile phase solvent for equilibration of the system flowing through the pump, tubing, and columns was preferably THF (non-inhibited, HPLC grade) used fresh after being filtered through 0.2 micron Teflon® filter.

In this embodiment, at least two high resolution GPC/SEC columns were placed in series (100Å, 3 micron, 300 x 7.5 mm) with a 3 micron column guard (50 x 7.5 mm). All columns and guards were connected to the HPLC system using minimum lengths of 0.17 mm i.d. stainless steel tubing. The columns were preferably maintained at 40°C, and the fluorescence detector preferably set at 220 for excitation and 345 for emission, and a UV detector was preferably set at 230 nm for diode array detection (or 245 nm for variable wavelength detection). The detector settings for the ELSD detector may vary among different detectors, and these were optimized for each detector.

The HPLC system was calibrated using 5-7 calibration solutions of an appropriate gasoline additive package in non-additized base fuel. A sample of "blank" non-additized base fuel was also prepared following the procedure described above to record a zero point on the calibration curve, as shown in Figure 1.

The blank and calibration samples were placed on a tray in the HPLC system, and 100 microliters from each vial was injected and eluted through the HPLC system over 70 minutes. The HPLC equipment was programmed to inject a calibration sample as a control after each sequence of 10-15 fuel samples to insure accuracy and precision for the analysis. Depending on the nature of the additives in the package, an aliquot of 100 µl of DMA, or a solvent of similar or higher polarity, was injected through the analytical system after each fuel sample to maintain the optimal condition of the column and reduce undesirable sample-column interactions.

Each sample then passed through a series of detectors that included a UV/VIS detector, a fluorescence detector, and optionally, a refractive index detector (RID) or an evaporative light scattering detector (ELSD).

Chromatograms from a standard were obtained for each detector using a calibration sample allow identification and quantitation of unknown samples, based on the peak area produced by each detector for known and unknown additive packages. The ratios of signals and responses for each detector were unique for each additive package analyzed, and were used to identify an unknown additive package-but also were used to check for contamination of a fuel sample with other GPAs. After the identification was achieved by processing the known and unknown samples, quantitation of additive package concentration was performed using the standard analytical software associated with the array of detectors that produced reproducible responses.
Figure 1, below, demonstrates a representative calibration curve obtained from the fluorescence detector (FLD) used in the analytical technique of the present invention and demonstrates reliable quantitation for GPA levels in the range of 0.05 to 0.75 mg/ml. Similar calibration curves were obtained for the other detectors in the experiment.

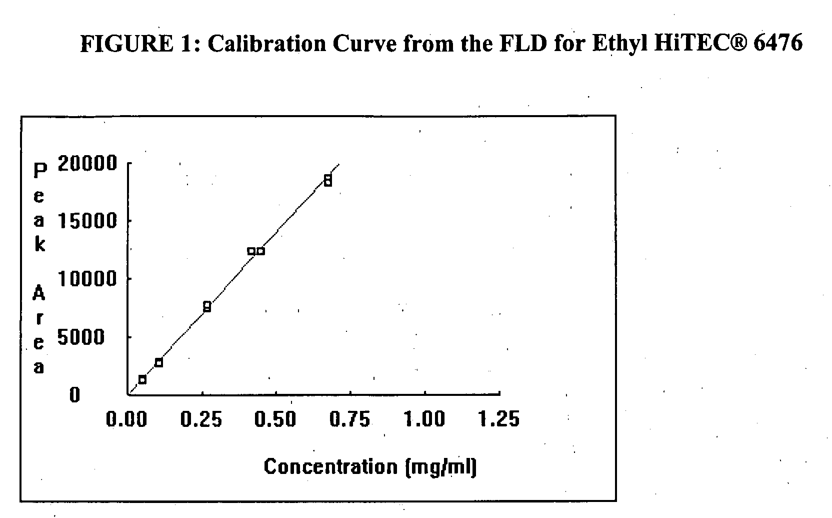

### EXAMPLE 2

In this experiment, a database of detector curves for many known additive packages was established for comparison to unknown samples. Such comparisons were achieved visually or by computerized programs.

A GPA in Fuel Sample A generated responses in two detectors, as shown in Figure 2 below graphing time in minutes on the horizontal axis and the intensity of response in milliamperes on the vertical axis. The upper curve was obtained using a fluorescence detector (FLD) and the lower curve was generated using a UV/VIS detector. Together these peaks contributed to a fingerprint of the additive package that enabled its identification. Using this fingerprint, Sample A's identity was determined by matching the detectors signals to those of a standard within the array of known packages.

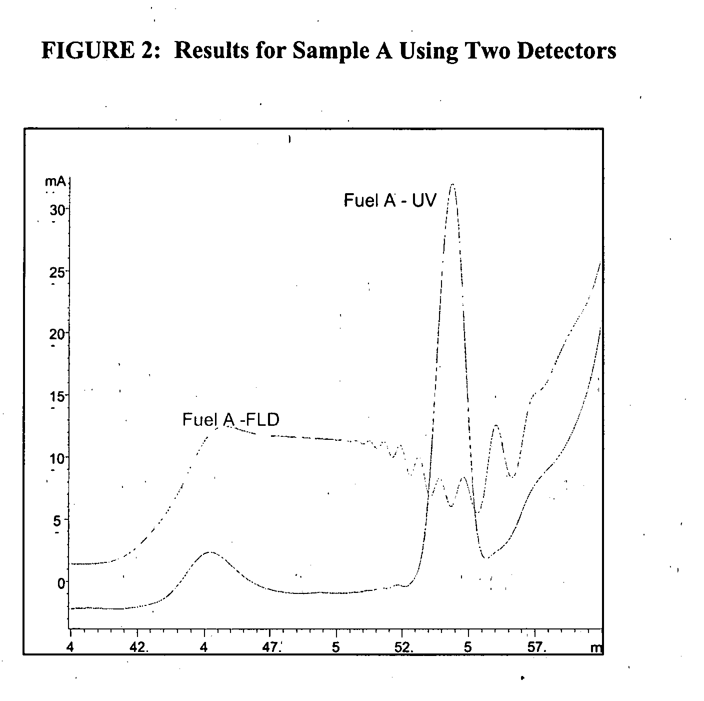

In this manner, the method of the present invention allows the investigator to determine the amount of a specific additive package present in a hydrocarbon matrix, and from that information determine if mixing or cross-hauling has occurred. The amount of the treat rate for each additive package allows the investigator to also determine the probable source of cross-hauling (e.g., fuel company, or additive supplier).

Thus, the present invention also provides a method for determining cross-hauling of fuel, comprising:
(i) delivering a sample of fuel to an analytical system comprising at least one separation means and at least one detector means, whereby at least one signal is generated by the analytical system; and
(ii) comparing signals from known standards of additive packages in hydrocarbon matrices to the at least one signal from the sample, to thereby determine the identity and/or concentration of any additive packages in the sample.

This invention is susceptible to considerable variation in its practice. Accordingly, this invention is not limited to the specific exemplifications set forth hereinabove. Rather, this invention is within the spirit and scope of the appended claims, including the equivalents thereof available as a matter of law.

The patentees do not intend to dedicate any disclosed embodiments to the public, and to the extent any disclosed modifications or alterations may not literally fall within the scope of the claims, they are considered to be part of the invention under the doctrine of equivalents.

## Claims

1. A method for determining the identity of an additive package or a mixture of additive packages, in a liquid hydrocarbon matrix, comprising:
(i) delivering a sample of the liquid hydrocarbon matrix to an analytical system comprising at least one separation means and at least one detector means, whereby at least one signal is generated by the analytical system; and
(ii) comparing signals from a standard of a known additive package in a hydrocarbon matrix to the at least one signal from the sample, to thereby determine the identity of an additive package in the sample.

2. The method of claim 1, wherein said hydrocarbon matrix is selected from group consisting of fuels, oils, naphtha, gasoline, kerosene, diesel fuels, jet fuels, turbine combustion fuel oils, gas oils, transmission fluids, and hydraulic fluids.

3. The method of claim 1, wherein the additive package is selected from the group consisting of fuel additives, components, and concentrates; lubricant additives, components, and concentrates; transmission fluid additives, components, and concentrates; and hydraulic fluids, components, and concentrates.

4. The method of claim 1, wherein delivering a sample to the analytical system further comprises filtering the sample through a 0.01 to 0.5 micron filter.

5. The method of claim 1, wherein delivering a sample to the analytical system further comprises filtering the sample through a 0.1 to 0.2 micron filter.

6. The method of claim 1, wherein a first solvent is used as a mobile phase in the analytical system and said first solvent is selected from the group consisting of toluene, ethyl acetate, tetrahydrofuran (THF), chloroform, methyl ethyl ketone (MEK), dichloromethane, dichloroethane, acetone, o-dichlorobenzene (o-DCB), trichlorobenzene (TCB), m-cresol, and o-chlorophenol (o-CP).

7. The method of claim 1, wherein a first solvent is used as a mobile phase in the analytical system and said first solvent is tetrahydrofuran (THF).

8. The method of claim 1, wherein an injection of a second solvent is used to optimize conditions in the analytical system and said second solvent is selected from the group consisting of pyridine, dimethyl acetamide (DMA), n-methyl pyrolidone (NMP), dimethyl sulphoxide (DMSO), and dimethyl formamide (DMF).

9. The method of claim 1, wherein an injection of a second solvent is used to optimize conditions in the analytical system and said second solvent is dimethyl acetamide (DMA).

10. The method of claim 1, wherein the analytical system operates using a mobile phase set at a flow rate between 0.05 and 2.5 ml/min.

11. The method of claim 1, wherein the analytical system operates using a mobile phase set at a flow rate of about 0.6 ml/min.

12. The method of claim 1, wherein the analytical system operates using a mobile phase set at a flow rate of about 0.3 ml/min.

13. The method of claim 1, wherein the separation means comprises at least one gel permeation chromatography size-exclusion (GPC/SEC) column.

14. The method of claim 1, wherein the separation means comprises at least two high-resolution low-pore size GPC/SEC columns.

15. The method of claim 1, wherein the separation means comprises at least two (100Å, 3 micron, 300x75mm) GPC/SEC columns.

16. The method of claim 1, wherein the detector means comprises at least one detector selected from the group consisting of a UV/VIS detector, a fluorescence detector, a refractive index detector (RID), an evaporative light scattering detector (ELSD), and a mass spectrophotometer detector (MSD).

17. The method of claim 1, wherein the detector means comprises a UV/VIS detector.

18. The method of claim 1, wherein the detector means comprises a UV/VIS detector and a fluorescence detector.

19. The method of claim 1, wherein the detector means comprises a UV/VIS detector, a fluorescence detector, and an RID.

20. The method of claim 1, wherein the detector means comprises a UV/VIS detector, a fluorescence detector, an RID, and an MSD.

21. The method of claim 1, wherein the detector means comprises a UV/VIS detector, a fluorescence detector, and an ELSD.

22. The method of claim 1, wherein the detector means comprises a UV/VIS detector, a fluorescence detector, an ELSD, and an MSD.

23. A method for determining the concentration of an additive package or a mixture of additive packages, in a liquid hydrocarbon matrix, comprising:
(i) delivering a sample of the liquid hydrocarbon matrix to an analytical system comprising at least one separation means and at least one detector means, whereby at least one signal is generated by the analytical system; and
(ii) comparing signals from a standard of a known additive package in a hydrocarbon matrix to the at least one signal from the sample, to thereby determine the identity of an additive package in the sample.

24. The method of claim 23, wherein said hydrocarbon matrix is selected from group consisting of fuels, oils, naphtha, gasoline, kerosene, diesel fuels, jet fuels, turbine combustion fuel oils, gas oils, transmission fluids, and hydraulic fluids.

25. The method of claim 23, wherein the additive package is selected from the group consisting of fuel additives, components, and concentrates; lubricant additives, components, and concentrates; transmission fluid additives, components, and concentrates; and hydraulic fluids, components, and concentrates.

26. The method of claim 23, wherein delivering a sample to the analytical system further comprises filtering the sample through a 0.01 to 0.5 micron filter.

27. The method of claim 23, wherein delivering a sample to the analytical system further comprises filtering the sample through a 0.1 to 0.2 micron filter.

28. The method of claim 23, wherein a first solvent is used as a mobile phase in the analytical system and said first solvent is selected from the group consisting of toluene, ethyl acetate, tetrahydrofuran (THF), chloroform, methyl ethyl ketone (MEK), dichloromethane, dichloroethane, acetone, o-dichlorobenzene (o-DCB), trichlorobenzene (TCB), m-cresol, and o-chlorophenol (o-CP).

29. The method of claim 23, wherein a first solvent is used as a mobile phase in the analytical system and said first solvent is tetrahydrofuran (THF).

30. The method of claim 23, wherein an injection of a second solvent is used to optimize conditions in the analytical system and said second solvent is selected from the group consisting of pyridine, dimethyl acetamide (DMA), n-methyl pyrolidone (NMP), dimethyl sulphoxide (DMSO), and dimethyl formamide (DMF).

31. The method of claim 23, wherein an injection of a second solvent is used to optimize conditions in the analytical system and said second solvent is dimethyl acetamide (DMA).

32. The method of claim 23, wherein the analytical system operates using a mobile phase set at a flow rate between 0.05 and 2.5 ml/min.

33. The method of claim 23, wherein the analytical system operates using a mobile phase set at a flow rate of about 0.6 ml/min.

34. The method of claim 23, wherein the analytical system operates using a mobile phase set at a flow rate of about 0.3 ml/min.

35. The method of claim 23, wherein the separation means comprises at least one gel permeation chromatography size-exclusion (GPC/SEC) column.

36. The method of claim 23, wherein the separation means comprises at least two high-resolution low-pore size GPC/SEC columns.

37. The method of claim 23, wherein the separation means comprises at least two (100Å, 3 micron, 300x75mm) GPC/SEC columns.

38. The method of claim 23, wherein the detector means comprises at least one detector selected from the group consisting of a UV/VIS detector, a fluorescence detector, a refractive index detector (RID), an evaporative light scattering detector (ELSD), and a mass spectrophotometer detector (MSD).

39. The method of claim 23, wherein the detector means comprises a UV/VIS detector.

40. The method of claim 23, wherein the detector means comprises a UV/VIS detector and a fluorescence detector.

41. The method of claim 23, wherein the detector means comprises a UV/VIS detector, a fluorescence detector, and an RID.

42. The method of claim 23, wherein the detector means comprises a UV/VIS detector, a fluorescence detector, an RID, and an MSD.

43. The method of claim 23, wherein the detector means comprises a UV/VIS detector, a fluorescence detector, and an ELSD.

44. The method of claim 23, wherein the detector means comprises a UV/VIS detector, a fluorescence detector, an ELSD, and an MSD.

45. A method for determining cross-hauling of fuel, comprising:
(i) delivering a sample of fuel to an analytical system comprising at least one separation means and at least one detector means, whereby at least one signal is generated by the analytical system; and
(ii) comparing signals from a standard of a known additive package in a hydrocarbon matrix to the at least one signal from the fuel sample, to thereby determine the identities and/or concentrations of all additive packages in the fuel sample.

46. The method of claims 1, 23, or 45 wherein the additive package comprises a succinimide dispersant.
